# EUROPEAN PATENT SPECIFICATION

(11) **EP 0 588 855 B1**
(45) Date of publication and mention of the grant of the patent: **08.01.1997**
(21) Application number: 92911535.0
(22) Date of filing: 05.06.1992
(51) Int. Cl.: C08B 37/10, A61K 31/725

(54) **ACYLATED HEPARINS AS INHIBITORS OF THE REPLICATION OF RETROVIRUSES**
Acylierte Heparine als Inhibitoren der Replikation von Retroviren
HEPARINES ACYLEES UTILISEES COMME INHIBITEURS DE LA REPLICATION DE RETROVIRUS

(30) Priority: 14.06.1991 IT MI91164
(43) Date of publication of application: 30.03.1994
(73) Proprietor: ITALFARMACO S.p.A., 20126 Milano (IT)
(72) Inventor: CARAMAZZA, Ida, I-20099 Sesto San Giovanni (IT); ZOPPETTI, Giorgio, I-20099 Sesto San Giovanni (IT); MODENA, Daniela, I-20099 Sesto San Giovanni (IT); SICCARDI, Antonio, G., I-20099 Sesto San Giovanni (IT); BERETTA, Alberto, I-20099 Sesto San Giovanni (IT); LOPALCO, Lucia, I-20099 Sesto San Giovanni (IT)
(74) Representative: Minoja, Fabrizio
(86) International application number: EP9201261
(87) International publication number: WO9222588

(56) References cited:
- EP-A- 0 293 826
- WO-A-90/03791
- US-A- 3 118 816

## Description

The subject of the present invention is N-acylated heparins that are capable of inhibiting the replication of retroviruses, in particular that of the human immunodeficiency virus (HIV).

These N-acylated heparins have a level of acylation, defined as the percentage ratio of the number of N-acylated residues to the number of N-sulphate groups present in the original heparin, of between approximately 10% and approximately 100%. They are prepared from heparins with molecular weights ranging between approximately 1,000 and approximately 30,000 Daltons, in other words commercial heparins of extractive origin, or fractions or fragments of such heparins.

The subject of the present invention is therefore heparins that have been N-acylated with residues derived from aliphatic monocarboxylic acids with between 3 and 20 carbon atoms, such as propionic, butyric, caproic, caprylic, caprinic, lauric, myristic, palmitic, stearic, crotonic, oleic, elaidinic, stearolic, tetrolic, arachic and similar acids, or with residues of aliphatic dicarboxylic acids containing between 3 and 10 carbon atoms, such as malonic, succinic, dimethylmalonic, glutaric, suberic, azelaic and sebacic acids. These mono-or di-carboxylic acids can also be substituted by one or more hydroxyl, amine or acylamine groups, to give, for example, heparins that are N-acylated with residues of lactic, hydracrylic, tartaric, tartronic, malic, aspartic, glutamic, N-acetylaspartic, N-acetylglutamic and similar acids.

N-succinylated heparins containing between 25 and 35% of succinic residues are described in US Patent No. 3,118,816, and heparins with a level of acylation of approximately 100% are known from GB Patent No. 2,078,768.

The inhibition of HIV replication by sulphated polysaccharides has been the subject of in-depth studies for some time (Lancet, 1, 1379, 1987; Biochem. Pharmacol. 37, 2987, 1988; Proc. Natl. Acad. Sci. U.S.A. 85, 6132, 1988). The polysaccharides that have been considered so far mainly belong to two classes: on the one hand, polysaccharides of bacterial or vegetable origin that are sulphated either naturally or artificially, such as carrageenans, pentosan sulphate, dextran sulphate, xylan sulphate (EP-A-0,406,685), and on the other hand, heparins or heparin fragments or fractions (EP-A-0,355,905). The former are believed to have the advantage of having little or no anticoagulant activity but little is known about their possible toxic effects, whereas the latter interfere significantly with the processes of blood coagulation but have the advantage of not carrying any unknown risks as regards their therapeutic use since they have already been used in medical practice for some considerable time.

The therapeutic value of non-heparin sulphated polysaccharides was recently explained on the basis of their ability to prevent the glycoprotein gp120 expressed on the surface of infected cells from interacting with the CD4 receptor of healthy cells, thereby inhibiting the subsequent formation of specific cell structures (syncytia) that play a primary role in the spread of the infection (J. Acquired Immun. Deficiency Syndromes, 3, 493, 1990).

It has now been found that the N-acylated heparins described above are highly effective in inhibiting the replication of retroviruses, in particular that of HIV, produce few, if any, signs of toxicity and have virtually no anticoagulant or antithrombotic activity.

The N-acylated heparins that are the subject of the present invention are obtained by a process that comprises N-desulphation of the initial heparins by known methods and their subsequent acylation by means of reaction with the chosen acid or one of its functional derivatives.

The N-desulphation is achieved in particular by heating solutions of the chosen heparin in the form of the corresponding heparinic acid to temperatures of between 30 and 60°C for approximately 24 hours. The level of N-desulphation and therefore of N-acylation that can be obtained depends on the reaction temperature used. Thus, for example, a temperature of approximately 55-60°C for 24 hours produces a level of N-desulphation of over 95%, whereas a temperature of not more than approximately 35°C gives a level of N-desulphation of approximately 30%. Temperatures between these two values obviously give N-desulphation values between the two limits given above. The subsequent N-acylation reaction is carried out using preferably, as the acylating agent, a symmetrical or mixed anhydride of the chosen acid. The reaction is effected for the most part at ambient temperature, with the pH of the solution being kept at between approximately 5 and approximately 7 by the addition of a suitable alkaline agent. The final product is thus obtained in the form of a salt, preferably the salt of an alkali or alkaline earth metal, which can be further purified or converted into another salt, preferably the salt of an alkali or alkaline earth metal, by methods known to experts in the subject.

By way of example, we describe below the preparation of a number of heparins with varying levels of N-succinylation, obtained by starting with heparins of different molecular weights.

### EXAMPLE 1

### Synthesis of N-succinylated heparin (100%)

### a) Preparation of N-desulphated heparin.

The N-desulphated heparin was prepared using a modified version of the method already described (Lloyd et al., Biochem. Pharm., vol. 30, 637-648, 1971). An aqueous solution containing 1.1 g porcine sodium heparin with a mean molecular weight of 13,600 D, with anti-Xa activity of 206 units/mg (chromogenic) and APTT activity of 202 units/mg, is percolated through a column thermostatically maintained at 4°C containing 50 ml Dowex® 50 W X 8H cationic resin. The eluate (50 ml) containing the heparinic acid has a pH of approximately 1.2. The solution thus obtained is placed in a flask and kept at 55°C for 24 hours, with gentle stirring and continuous monitoring of the temperature. The solution is then neutralised to pH 7 with 1 N NaOH at ambient temperature. The solution is then freeze-dried, giving the N-desulphated heparin in the form of a white powder. The level of N-desulphation is >95% according to ¹³C-NMR analysis (shifting of the C₁ signal of the aminosugar from 99.5 to 94 ppm and shifting of the C₂ signal from 60.5 to 66.8 ppm).

### b) Preparation of N-succinylated heparin.

The intermediate product obtained in a) above is dissolved in 100 ml water (1% solution) with stirring. The solution is neutralised to pH 7 with 0.1 N NaOH, then 2 g solid succinic anhydride are added in aliquots of 250 mg at a time, the entire procedure being carried out at ambient temperature. After each addition it is necessary to readjust the pH from 5 to 7 with 0.1N NaOH. At the end of the reaction the excess insoluble sodium succinate is filtered off. The clear solution thus obtained is treated with 3 volumes of absolute ethanol, to produce a precipitate. The precipitate is washed repeatedly with absolute ethanol. A solid, white precipitate is obtained. The precipitate is redissolved in water (5% solution). The solution is placed in a dialysis membrane (SPECTRAPOR® cut-off 8000 D) for 24 hours against 2 1 of water. At the end of this time, the intradialysis solution is freeze-dried to give the N-succinylated heparin in the form of a white powder, which has the characteristics set out in Table 1.

### EXAMPLE 2

### Synthesis of partially (50%) N-succinylated heparin

The procedure is the same as that described in Example 1 but uses for the succinylation the partially (>50%) N-desulphated heparin derivative which can be obtained by the method described in Example 1a, though in this case the reaction is carried out at 40°C for 24 hours. The level of desulphation of this product is checked by the ¹³C-NMR test as already described. The partially (50%) N-succinylated heparin in the form of a white powder has the characteristics set out in Table 1.

### EXAMPLE 3

### Synthesis of 100% N-succinylated LMW heparin

The procedure is the same as that described in Example 1, but uses 1 g of low molecular weight heparin (average molecular weight 5000 D), with anti-Xa activity of 97 units/mg and APTT activity of 74 units/mg (Calbiochem). In this case the N-succinylated derivative is purified from the excess succinate in a dialysis membrane (Spectrapor®) with a cut-off of 1000 D, again as already described in Example 1. After freeze-drying, the 100% N-succinylated LMW heparin is obtained in the form of a white powder, which has the characteristics set out in Table 1.

### EXAMPLE 4

### Synthesis of partially (50%) N-succinylated LMW heparin

The procedure is the same as that described in Example 2, and uses 1 g of the same low molecular weight heparin as used in the previous example. This yields 50% of N-succinylated LMW heparin with the characteristics set out in Table 1.

### EXAMPLE 5

### Synthesis of 100% N-succinylated VLMW heparin

The procedure is the same as that described in Example 1, but uses 1 g of very low molecular weight heparin (molecular weight <3000 D, anti-Xa activity: >50 units/mg; APTT activity: <45 units/mg) (Sigma). In this case the N-succinylated derivative is purified from the excess succinate by means of fractional precipitation, which is based on the selective formation of complexes with alkylammonium salts using the original method of Scott (Methods of Biochem. Anal., vol. 8, 145-197, 1960). In brief, the 5% solution of N-succinylated VLMW heparin is subjected to selective precipitation using 10% cetylpyridinium chloride in the absence of salts, in the cold (4°C); the mixture is left to stand for approximately 2 hours, then centrifuged at 4000 rpm for 20 minutes. The resulting precipitate is separated from the excess succinate that remains in solution. After being washed 3 times with hot; distilled water, the precipitate is redissolved hot (30-35°) in a 2M NaCl solution. Finally, the solution is treated with 3 volumes of absolute ethanol, causing the precipitation of the N-succinylated VLMW sodium heparin, which has the characteristics set out in Table 1.

### EXAMPLE 6

### Synthesis of partially (50%) N-succinylated VLMW heparin

The procedure is the same as that described in Example 2 and uses 1 g of the same very low molecular weight heparin used in the previous example. The N-succinylated derivative is purified from the excess succinate in the same way as described in Example 6. This yields 50% N-succinylated VLMW heparin, which has the characteristics and biological activity set out in Table 1.

### EXAMPLE 7

### Calcium salt of partially (50%) N-succinylated heparin

The product of Example 2 (1 g) is percolated through a column thermostatically maintained at 4°C and containing 50 ml DOWEX 50W-X8H⁺ cationic resin. The eluate containing the acid form of the intermediate product is titrated with a stoichiometric quantity of calcium carbonate (CaCO₃). The solution is then freeze-dried, yielding the partially (50%) N-succinylated calcium heparin in the form of a white powder, which has the characteristics set out in Table 1.

### Example 8

### Calcium salt of partially (50%) N-succinylated LMW heparin

The product of Example 4 is treated as described in Example 7 to obtain the calcium salt of the partially (50%) N-succinylated LMW heparin, which has the characteristics set out in Table 1.

### EXAMPLE 9

### Calcium salt of partially (50%) N-succinylated VLMW heparin

The product of Example 6 is treated as described in Example 7 to obtain the calcium salt of the partially (50%) N-succinylated VLMW heparin, which has the characteristics set out in Table 1.

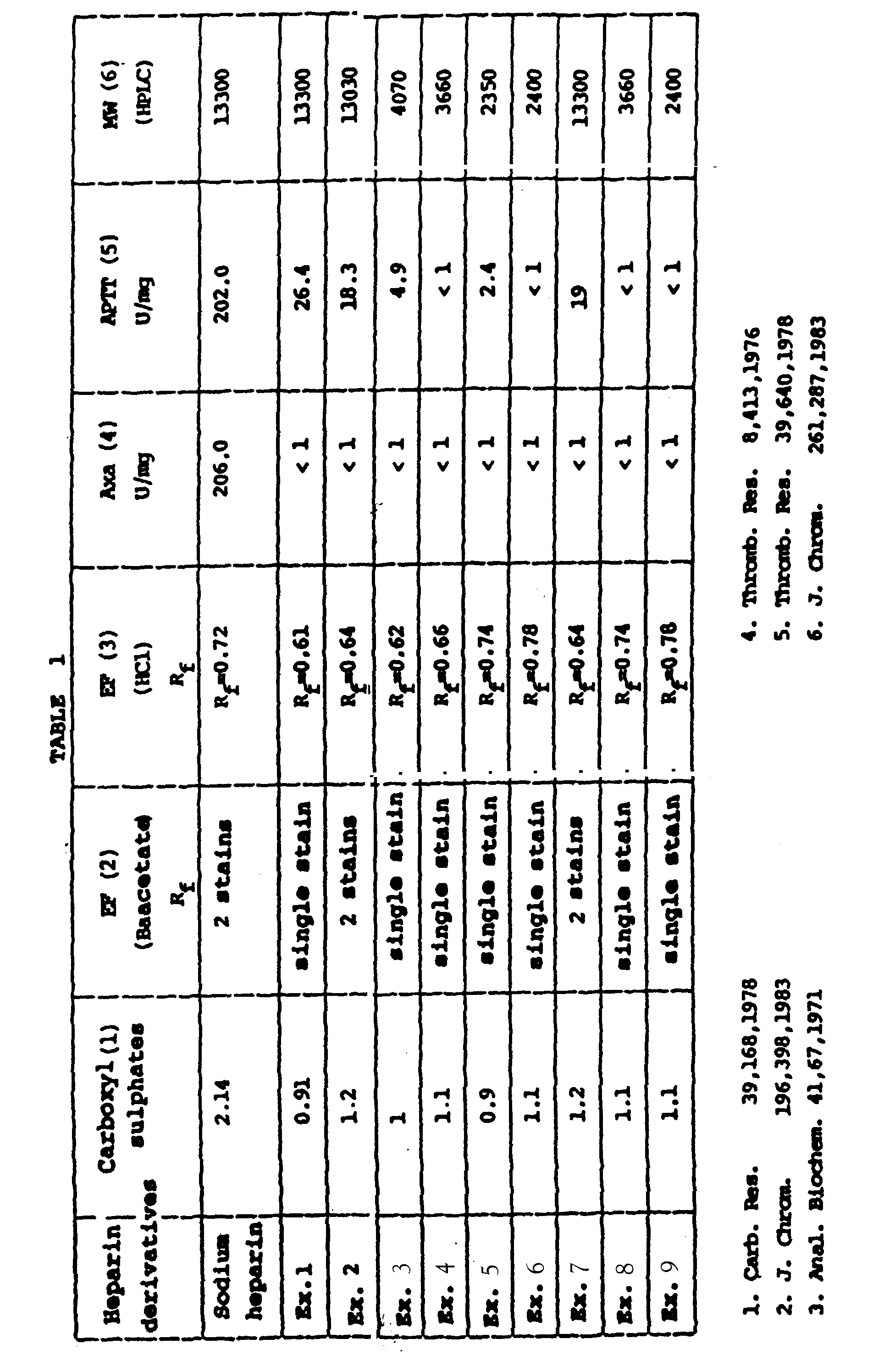

The antiviral activity of the compounds described above was evaluated with the aid of the syncytia formation inhibition test, using the method described below.

8E51 cells permanently infected with LAV+ (Lymphotropic Adenopathy Virus) and defective in the pol gene, and MOLT3 cells (CD4+ human T-lymphoblastoid line) cultivated in RPMI 1640 medium with 10% foetal calf serum in a concentration of 10⁶ cells/ml are incubated together in a ratio of 1:2 and distributed in Microtiter plates with 96 conical-bottomed wells. The heparin derivatives are added to the cells in the appropriate dilutions, then after 30 minutes' incubation at 37°C sedimentation is achieved by centrifuging. The pellet, incubated for a further 2.5 hours at 37°C, is transferred into flat-bottomed wells. The syncytia (10-100 times larger than the initial cells) are counted with an overhead microscope. In the control samples, it is possible to see some 100-200 syncytia in each well under the microscope.

The N-succinylated heparins that constitute the subject of the invention proved to be capable of inhibiting the formation of syncytia in concentrations of between 0.01 and 10 µg/well. Especially good results were obtained with the product as described in Example 3. According to preliminary data, results equivalent to those obtained with the syncytia are obtained in cytofluorimetric conditions, where the N-succinylated heparins compete only with the bond of the anti-gpl20 antibodies on the 8E51 cells. In view of their virtual lack of anticoagulant activity, the N-succinylated heparins according to the invention are proposed as selective antiviral agents that are substantially devoid of side effects.

For the envisaged therapeutic applications, the succinylated heparins according to the invention will be formulated in suitable pharmaceutical forms, using conventional methods and excipients, such as those described in Remington's Pharmaceutical Sciences Handbook, Mack Pub. Co., New York, U.S.A.. The heparins according to the invention will be administered for the most part parenterally, but the use of other administration routes, for example the oral route, is not ruled out, in which case appropriate salts would be used. The dosage will depend on various factors and will need to be established on an individual basis, but will generally be between 10 and 1000 mg of the compounds described above, given in one to four doses per day.

## Claims (Claims for the following Contracting State(s): AT, BE, CH, LI, DE, DK, FR, GB, IT, LU, MC, NL, SE)

1. Heparins N-acylated with residues of aliphatic monocarboxylic acids with between 3 and 20 carbon atoms or aliphatic dicarboxylic acids with between 3 and 10 carbon atoms, having molecular weight ranging between 1,000 and 30,000 daltons having a level of acylation, defined as the percentage ratio of the number of N-acylated residues to the number of N-sulfate groups present in the original heparin, of between 10% and 100% with the proviso that when heparins are acylated with succinic acid residues, the acylation degree is not between 25% and 35%.

2. Heparins according to claim 1 in which the mono- and di-carboxylic acids are substituted by one or more hydroxy, amine or acylamino groups.

3. Heparins according to claim 1 N-acylated with residues of succinic acid.

4. Process for the preparation of N-acylated heparins according to claims 1-3 from heparins, or their fractions, or their fragments, that are subjected to N-desulphation followed by N-acylation with a mono- or di-carboxylic acid or with their functional derivatives.

5. Pharmaceutical compositions with anti-HIV activity containing as active principle an N-acylated heparin according to claims 1-3.

## Claims (Claims for the following Contracting State(s): ES, GR)

1. A process for the preparation of heparins N-acylated with residues of aliphatic monocarboxylic acids with between 3 and 20 carbon atoms or aliphatic dicarboxylic acids with between 3 and 10 carbon atoms, having molecular weight ranging between 1,000 and 30,000 daltons having a level of acylation, defined as the percentage ratio of the number of N-acylated residues to the number of N-sulfate groups present in the original heparin, of between 10% and 100% with the proviso that when heparins are acylated with succinic acid residues, the acylation degree is not between 25% and 35%, which comprises the N-desulphation of heparins or fractions or fragments thereof followed by N-acylation with a mono-or dicarboxylic acid or with their functional derivatives.

## Patentansprüche (Patentansprüche für folgende(n) Vertragsstaat(en): AT, BE, CH, LI, DE, DK, FR, GB, IT, LU, MC, NL, SE)

1. Heparine, die mit Resten aliphatischer Monocarbonsäuren mit zwischen 3 und 20 Kohlenstoffatomen oder aliphatischen Dicarbonsäuren mit zwischen 3 und 10 Kohlenstoffatomen N-acyliert sind, mit einem Molekulargewicht im Bereich zwischen 1000 und 30000 Dalton, mit einem Acylierungsgrad, definiert als prozentuales Verhältnis der Anzahl von N-acylierten Resten zur Anzahl von N-Sulfatgruppen, die im ursprünglichen Heparin vorhanden sind, von zwischen 10 % und 100 %, mit der Maßgabe, daß, wenn die Heparine mit Succinsäureresten acyliert sind, der Acylierungsgrad nicht zwischen 25 % und 35 % liegt.

2. Heparine nach Anspruch 1, worin die Mono- oder Dicarbonsäuren mit einer oder mehreren Hydroxy-, Amin- oder Acylaminogruppen substituiert sind.

3. Heparine nach Anspruch 1, die mit Succinsäureresten N-acyliert sind.

4. Verfahren zur Herstellung der N-acylierten Heparine nach den Ansprüchen 1 bis 3 aus Heparinen, deren Fraktionen oder deren Fragmente, die der N-Desulfatisierung, gefolgt von der N-Acylierung mit einer Mono- oder Dicarbonsäure oder deren funktionellen Derivate unterworfen werden.

5. Pharmazeutische Zusammensetzungen mit Anti-HIV-Wirkung, die als aktiven Bestandteil N-acyliertes Heparin nach den Ansprüchen 1 bis 3 enthalten.

## Patentansprüche (Patentansprüche für folgende(n) Vertragsstaat(en): ES, GR)

1. Verfahren zur Herstellung von Heparinen, die mit Resten aliphatischer Monocarbonsäuren mit zwischen 3 und 20 Kohlenstoffatomen oder aliphatischen Dicarbonsäuren mit zwischen 3 und 10 Kohlenstoffatomen N-acyliert sind, mit einem Molekulargewicht im Bereich zwischen 1000 und 30000 Dalton, mit einem Acylierungsgrad, definiert als prozentuales Verhältnis der Anzahl von N-acylierten Resten zur Anzahl von N-Sulfatgruppen, die im ursprünglichen Heparin vorhanden sind, von zwischen 10 % und 100 %, mit der Maßgabe, daß, wenn die Heparine mit Succinsäureresten acyliert sind, der Acylierungsgrad nicht zwischen 25 % und 35 % liegt,
das umfaßt: N-Desulfatisierung der Heparine, deren Fraktionen oder deren Fragmente, gefolgt von der N-Acylierung mit Mono- oder Dicarbonsäuren oder deren funktionellen Derivate.

## Revendications (Revendications pour l'(les) Etat(s) contractant(s) suivant(s): AT, BE, CH, LI, DE, DK, FR, GB, IT, LU, MC, NL, SE)

1. Héparines N-acylées avec des restes d'acides monocarboxyliques aliphatiques ayant entre 3 et 20 atomes de carbone ou d'acides dicarboxyliques aliphatiques ayant entre 3 et 10 atomes de carbone, ayant un poids moléculaire compris entre 1 000 et 30,000 daltons et un niveau d'acylation, défini comme le rapport en pourcentage du nombre de restes N-acylés au nombre de groupes N-sulfates présent dans l'héparine d'origine, compris entre 10 % et 100 %, avec la condition que lorsque les héparines sont acylées avec des restes d'acide succinique, le degré d'acylation n'est pas compris entre 25 % et 35 %.

2. Héparines selon la revendication 1, dans lesquelles les acides mono- et di-carboxyliques sont substitués par un ou plusieurs groupes hydroxy, amine ou acylamino.

3. Héparines selon la revendication 1, N-acylées avec des restes d'acide succinique.

4. Procédé de préparation d'héparines N-acylées selon les revendications 1-3 à partir d'héparines, ou de leurs fractions, ou de leurs fragments, qui sont soumises à une N-désulfatation suivie par une N-acylation avec un acide mono- ou di-carboxylique ou avec leurs dérivés fonctionnels.

5. Compositions pharmaceutiques ayant une activité anti- VIH, contenant comme principe actif une héparine N-acylée selon les revendications 1-3.

## Revendications (Revendications pour l'(les) Etat(s) contractant(s) suivant(s): ES, GR)

1. Un procédé de préparation d'héparines N-acylées avec des restes d'acides monocarboxyliques aliphatiques ayant entre 3 et 20 atomes de carbone ou d'acides dicarboxyliques aliphatiques ayant entre 3 et 10 atomes de carbone, ayant un poids moléculaire compris entre 1 000 et 30,000 daltons et un niveau d'acylation, défini comme le rapport en pourcentage du nombre de restes N-acylés au nombre de groupes N-sulfates présent dans l'héparine d'origine, compris entre 10 % et 100 %, avec la condition que lorsque les héparines sont acylées avec des restes d'acide succinique, le degré d'acylation n'est pas compris entre 25 % et 35 %, qui comprend la N-désulfatation d'héparines, ou de fractions ou de fragments de celles-ci, suivie par une N-acylation avec un acide mono- ou di-carboxylique ou avec leurs dérivés fonctionnels.
